# EUROPEAN PATENT APPLICATION

(11) **EP 0 857 483 A1**
(43) Date of publication of application: **12.08.1998**
(21) Application number: 98300968.9
(22) Date of filing: 10.02.1998
(51) Int. Cl.: A61K 31/505

(54) **Substituted aminopyridines in the treatment of glucose metabolism disorders**

(30) Priority: 11.02.1997 US 798661
(71) Applicant: ELI LILLY AND COMPANY, Indianapolis, Indiana 46285 (US)
(72) Inventor: Steinberg, Mitchell Irvin, Indianapolis, Indiana 46260 (US); Yakubu-Madus, Fatima Emitsel, Indianapolis, Indiana 46256 (US)
(74) Representative: Hudson, Christopher Mark

(57) **Abstract**

The present invention provides a method of inhibiting glucose metabolism deterioration in a mammal by administering a substituted aminopyrimidine having the Formula 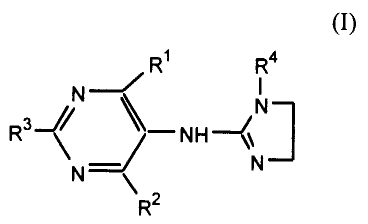 wherein
R¹, R², and R³ are, independently, hydrogen, halogen, C₁-C₄ alkoxy, C₁-C₄ alkylthio, C₁-C₄ alkyl, or C₃-C₅-cycloalky;
R⁴ is hydrogen, formyl, acetyl, propionyl or butyryl;
provided that R¹, R² and R³ are not all hydrogen at the same time;
or a pharmaceutically acceptable salt or solvate thereof.

## Description

Diabetes is a systemic disease characterized by hyperglycemia, hyperlipidemia, and hyperaminoacidemia. It is caused by a decrease in the secretion or activity of insulin or both and a decrease in the metabolic clearance and utilization of glucose and fatty acids. Diabetes is associated frequently with specific lesions of the microcirculation, neuropathic disorders, and a predisposition to atherosclerosis. The diabetic syndromes represent a diverse clinical spectrum manifesting a variety of genetic, immunologic, biochemical, and epidemiologic expressions for which precise etiologies have yet to be established.

Diabetes mellitus is an extremely prevalent disease striking approximately 16 million people in the Unites States alone with an estimated 100 million affected individuals worldwide. This year (1997) 625,000 people in the US will be diagnosed with diabetes, amounting to approximately one person every minute of every day.

Four major types of diabetes have been defined:
insulin-dependent diabetes mellitus (IDDM or Type I), non-insulin-dependent diabetes mellitus (NIDDM or Type II), gestational diabetes mellitus (GDM), and diabetes secondary to other conditions. Type I characteristically presents with prominent diabetes symptoms and extreme hyperglycemia. Type II can be diagnosed by the presence of the classical signs and symptoms of diabetes together with unequivocal hyperglycemia (blood glucose greater than 200 mg/dl); by fasting plasma glucose (FPG) greater than or equal to 140 mg/dl; or by venous plasma glucose greater than or equal to 200 mg/dl at 2 hours after a 75-g oral glucose challenge. Criteria for GDM vary but require an oral glucose challenge and measurement of post-load plasma glucose.

Insulin-dependent diabetes mellitus (Type I) is usually characterized by a relatively abrupt onset of symptoms, although present evidence suggests that its evolution may involve a longer antecedent period of autoimmune damage to the pancreatic β cells. Islet-cell antibodies are usually present at the onset of symptoms. Genetic factors related to histocompatibility antigens (human lymphocyte antigens) DR3 and DR4 are associated with the predisposition to humoral or cytotoxic immune activity directed against islet tissues. Environmental factors, particularly certain viral agents such as Coxsackie B, mumps, and rubella, are postulated to play an etiologic role in predisposed individuals.

Failure of insulin secretory capacity causes ketosis, a clinical feature that differentiates insulin dependent from non-insulin dependent forms of diabetes. Patients with insulin dependent (Type I) disease depend on insulin injection to prevent ketosis and to sustain health. Acute onset Type I diabetes occurs primarily in young patients, but a slower onset form may be seen at any age.

Noninsulin-dependent diabetes mellitus (Type II) is recognized as having a strong genetic basis, as evidenced by studies of identical twins and by familial transmission of diabetes in an autosomal dominant inheritance pattern. There is, however, no association with human lymphocyte antigen distributions, and islet-cell antibody is not present in the majority of patients initially diagnosed with NIDDM. Fasting and stimulated insulin responses vary considerably in patients with Type II disease. In some, insulin may be required to correct hyperglycemia. Patients with Type II disease are ketosis-resistant under normal conditions, but may exhibit ketosis during severe stress or infection. Environmental factors, such as obesity, play a role in inducing or worsening glucose tolerance in these patients. Within the Type II diabetic population is a subgroup of latent Type I subjects characterized by the presence of islet-cell antibodies, slowly progressive β-cell deterioration, and a long latency period.

While not necessarily mutually exclusive, pathophysiologic alterations are generally of two forms in patients with Type II diabetes: (1) a decreased insulin secretory response to the glucose stimulus characterized by reduced acute insulin release, and decreased total insulin secretion relative to the blood glucose concentration; or (2) reduced sensitivity or responsiveness to insulin related to decreased insulin-receptor activity or to pre- or postreceptor defects. The latter conditions involve a disturbance in glucose transport across cell membranes, a decrease in cellular processing of substrate resulting from reduced action of the insulin-receptor complex and its mediator systems within the cell, or a reduction in the delivery of hormone or substrate to metabolically active tissues.

Two major sites of insulin resistance are defined in Type II diabetes mellitus. In the fasting state, the overproduction of glucose identifies the liver as a site of insulin resistance. This is reflected in an increased hepatic glucose output in the postabsorptive state leading to a rise in fasting plasma glucose concentrations. This increase in hepatic glucose output is suppressed by insulin to a variable extent. In more severe diabetes, the ability of insulin to inhibit hepatic glucose output is reduced. In the fed state, the primary site of insulin resistance is muscle, demonstrated by a 50% reduction in glucose uptake by peripheral tissues of patients with diabetes during insulinglucose clamp studies. Muscle is the primary tissue that requires insulin for glucose uptake.

Type II diabetes, which occurs in 80 to 85% of the diabetic population, is usually encountered in adults, but may occur in young patients. Approximately 80% of Type II patients are obese, although the percentages have ranged from 60 to 90% in some studies. Patients with Type II diabetes may respond to weight reduction, dietary management, exercise, and medications. Insulin injections are used for those patients who require exogenous insulin during periods of hyperglycemia.

Type II diabetes mellitus is currently treated by a combination of behavioral, physical, dietary, and pharmacologic interventions. Specific diabetic meal planning and a graded exercise program are the first modalities instituted in all patients diagnosed with diabetes. This approach rarely succeeds given the extensive behavioral and societal changes that must be rigorously adhered to indefinitely. Indeed, diet therapy alone is effective in controlling blood glucose in less than 10% of patients.

The sulfonylurea class of compounds have been a foundation in the treatment of Type II diabetes mellitus for decades. However, between 20 - 30% of patients will fail to have glycemic improvement when this agent is started and an additional 10% of patients each year will become non-responders. Furthermore, an additional 5 - 10% will develop secondary failure each year such that after 10 years of therapy, less than 30% of those initially treated with sulfonylureas will have adequate control of blood glucose. Finally, these agents as a class, cause significant increases in weight that tend to exacerbate insulin resistance and hyperglycemia.

Metformin (a biguanide) and acarbose (an α-glucosidase inhibitor), also improve glycemic control but both are less effective than sulfonylureas. Furthermore, both compounds are associated with gastrointestinal side effects that may limit compliance. Recently, troglitazone, a thiazolidinedion has been approved for the treatment of NIDDM.

When oral therapy fails, the vast majority of patients require parenteral treatment with insulin. Although highly effective, insulin usage requires subcutaneous injections and refrigeration and is associated with an increased incidence of hypoglycemic events. Appropriate therapy invariably leads to weight gain and hyperinsulinemia, a circumstance that may contribute to further insulin resistance, resulting in greater changes in the abnormalities associated with the Metabolic Syndrome.

The Metabolic Syndrome or Syndrome X, along with impaired glucose homeostasis, includes: obesity; hypertension; and hypertriglyceridemia and reduced high density lipoprotein-cholesterol. For many years, a common link between these abnormalities has been sought. Several mechanisms have been proposed as the underlying etiology of the Metabolic Syndrome including hyperinsulinemia, abnormal regulation of muscle blood flow, excess sympathetic tone, excess circulating free fatty acids and abnormal lipolysis, and changes in baroreceptor function. All of these changes are tied to insulin resistance which itself can be explained by an abnormality in skeletal muscle glucose utilization or extraction. Analogous to the Metabolic Syndrome, the specific cause of myogenic insulin resistance is not fully understood and has been explained as one or several abnormalities. These include impaired delivery of glucose to the muscle, abnormal glucose transport into the tissue, and aberrant intracellular utilization of glucose. In the early phases of impaired glucose tolerance, hyperinsulinemia compensates for insulin resistance and glucose homeostasis is maintained. Over time, a reduction in the ability of the pancreatic islets to sustain levels of insulin secretory capacity that are capable of overcoming insulin resistance leads to the pathognomonic finding of NIDDM: hyperglycemia.

Given the large percentage of patients with NIDDM who are not effectively treated on current modalities or oral therapy, there is intense interest in compounds aimed against novel cellular and molecular targets that improve parameters of the Metabolic Syndrome and glycemic control. Thiazolidinediones are the first new class of drug to treat diabetes in more than 25 years. Although the glycemic efficacy of these compounds are limited and some concerns regarding overall safety still exist, there is, nonetheless, a great deal of excitement over their potential use. Both health care practitioners and patients understand that a vast need exists for new oral therapies in the prevention and treatment of glucose metabolism deterioration.

Sympathetic outflow has been the target of several anti-hypertensive medications. Many of these agents (α-methyldopa, guanabenz, guanfacine, moxonidine and clonidine) have been proposed to act via central α₂-adrenergic receptors thereby decreasing CNS sympathetic outflow. Although clonidine has potent α₂-adrenergic activity, its anti-hypertensive efficacy as well as the efficacy of moxonidine has been more closely correlated to its ability to activate a separate family of central nervous system receptors, the imidazoline-1 (I₁) receptor.

Although both moxonidine and clonidine may reduce blood pressure via the I₁ receptor, the potent α-adrenergic activity of clonidine likely diminishes its ability to alter glucose metabolism. That is, peripheral α-adrenergic stimulation leading to changes in perfusion of insulinresponsive tissues may counterbalance any beneficial effect of decreases in central sympathetic outflow. A significant portion of the side effect profile of clonidine is related to peripheral α-adrenergic activity such as sedation. In contrast, moxonidine has a significantly higher affinity for the I₁ receptor relative to the α₂ receptor. With lower α-adrenergic activity, moxonidine may represent a new therapeutic class of hypoglycemic or anti-hyperglycemic agents that elicit changes in glucose metabolism by selective central I₁ activation. Indeed, several lines of pre-clinical evidence support the hypothesis that moxonidine can improve hyperglycemia in Type II diabetes mellitus.

In EP 0 689 837, the use of moxonidine and its nontoxic acid salts for the treatment of glucose metabolism disturbances accompanied by hyperglycemia is described; that is, treatment of the hyperglycemic sequela.

It has now been discovered that certain substituted aminopyrimidines as defined below, and the pharmaceutically acceptable salts and solvates thereof can be used to inhibit glucose metabolism deterioration in mammals having glucose metabolism abnormalities and mammals at risk for developing glucose metabolism abnormalities, prior to the onset of hyperglycemia, thereby preventing or delaying the onset of glucose metabolism abnormalities, and preventing or delaying deterioration in glucose metabolism if a glucose metabolism abnormality exists; that is, treatment of one or more etiologies causing, or at risk for causing glucose metabolism deterioration.

Other objects, features and advantages of the present invention will become apparent from the subsequent description and the appended claims.

The present invention provides a method of inhibiting glucose metabolism deterioration in a mammal having a glucose metabolism abnormality or at risk for developing a glucose metabolism abnormality (treating one or more etiologies of glucose metabolism deterioration) prior to the onset of hyperglycemia comprising administering to said mammal an effective dose of a substituted aminopyrimidine, or a pharmaceutically acceptable salt or solvate thereof, having the formula: 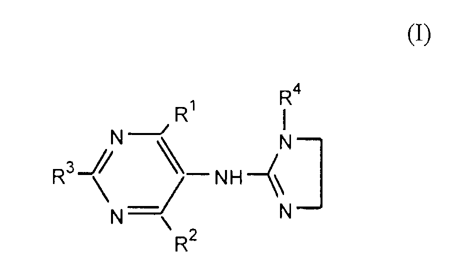 wherein
R¹, R², and R³ are, independently, hydrogen, halogen, C₁-C₄ alkoxy, C₁-C₄ alkylthio, C₁-C₄ alkyl, or C₃-C₅-cycloalkyl;
R⁴ is hydrogen, formyl, acetyl, propionyl or butyryl;
provided that R¹, R² and R³ are not all hydrogen at the same time;

The method of this invention is practiced by administering to a mammal having a glucose metabolism abnormality or at risk for developing a glucose metabolism abnormality an effective dose of a substituted aminopyrimidine of Formula I or a pharmaceutically acceptable salt or solvate thereof, preferably in a pharmaceutical formulation. It is necessary that the mammal being treated have functioning pancreatic beta cells.

The compound 4-chloro-*N*- (imidazoline-2-ylidene) -6-methoxy-2-methyl-5-pyrimidineamine (moxonidine) is known and described in U.S. Patent No. 4,323,570 which is incorporated herein by reference in its entirety, and is the preferred compound of the present invention.

The compounds of Formula I are prepared by reacting the corresponding 5-aminopyrimidines of Formula II 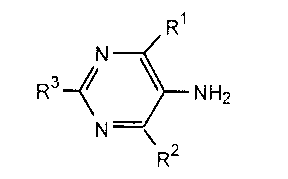 wherein R¹, R² and R³ are as defined above for Formula I with 1-acyl-imidazolidin-2-ones of Formula III 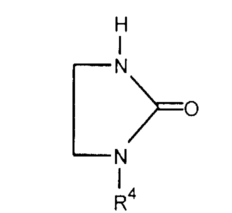 wherein R⁴ is formyl, acetyl, propionyl, or butyryl. Compounds of Formulas II and III are well-known or are synthesized in accordance with known methods of preparation.

The term "halogen" means chloro, bromo, fluoro and iodo. Among these halogens, chloro and bromo are preferred and most preferred is chloro.

The cycloalkyl substituents which are particularly suitable are cyclopropyl, cyclobutyl, or cyclopentyl, and C₁-C₄ alkyl derivatives thereof.

The aminopyrimidines substituted by alkyl or cycloalkyl groups in the 2 position are synthesized from the correspondingly substituted imino ethers. Starting with the nitriles, amidines are cyclized with malonic esters to form the hydroxypyrimidine derivatives. The desired substituted halogenated aminopyrimidines are formed by halogenation with exchange of hydroxy groups and the introduction of the amino group over the nitrile group.

If aminopyrimidines substituted in the 2 and 4 position are desired, synthesis begins by halogenation of the corresponding 6-uracils and introduction of the amino group as previously described.

The reaction between the aminopyrimidines and the acylimidazolidinones can be carried out in the presence of phosphorous oxychloride between about 20° C. and the boiling point of the phosphorous oxychloride. The reaction takes between 3 and 70 hours. It is preferred that an excess of the phosphorous compound be used so that it also serves as a solvent. However, inert organic solvents or mixtures thereof may also be used. Most preferable, in the event that phosphorous oxychloride is used alone, is a reaction of time of approximately 50 hours at temperatures between 50° and 100° C. After removing the excess phosphorous oxychloride with water or by means of an aqueous alkaline medium (e.g. sodium carbonate solution or lye), the reaction product is converted to the acyl derivatives, preferably at reduced temperature.

For the compounds in which R⁴ is hydrogen, the acyl group can be split off by the use of mineral acids, organic acids, and/or alkaline reagents. Advantageously, acetic acid, lye, sodium carbonate, alkali alcoholates or amines may be used. It is especially preferred to remove the acetyl group by heating with water or aliphatic alcohols. Methanol is particularly useful for this purpose. This means of removal is particularly gentle and, for that reason, desirable.

It is also possible to convert the compounds wherein R⁴ is hydrogen to the acyl form by reacting in a known manner with a recognized acylating agent. In this manner, new 1-acylimidazolines are obtained. Typical acylating agents are acid anhydrides and acid chlorides in the presence of bases such as pyridine. In this manner, such acyl groups as formyl, acetyl, propionyl, or butyryl may be introduced.

The corresponding alkoxy and alkylthio compounds can also be obtained from the halogenated compounds by substitution of the halogen atoms under suitable reaction conditions. The halogen substituents on the pyrimidine radical can be converted to alkoxy groups by the use of alcohols or alcoholates, preferably at a temperature between O° C. and the boiling point of the alcohol or alcoholates. Depending upon the reaction conditions, one or more alkoxy groups can be substituted for a corresponding number of halogen atoms.

If desired, the reaction can be carried out so that, by a suitable selection of the conditions (temperature, reaction time, concentration of reactants, etc.) at least one halogen atom bonded to the pyrimidine radical is still available. If this is done, the alkylthio compounds according to the invention can be prepared. Moreover, under more extreme conditions of higher temperature, longer reaction time, and higher alcohol or alcoholate concentration, additional halogen atoms can be converted. Also, separation of the R⁴ acyl group is possible.

The reactions in question are carried out either at room temperature or higher temperatures, preferably in alcohols or diluted alcohol solutions. Suitable alcohols (and alcoholates) are these having 1 to 4 carbon atoms, especially methanol and ethanol.

It is also possible to produce compounds of Formula I wherein R¹ is halogen, hydrogen, alkoxy, alkylthio, or alkyl with 1 to 4 carbon atoms or a cycloalkyl group with 3 to 5 carbon atoms; R² is alkoxy having 1 to 4 carbon atoms; R³ is hydrogen, alkoxy, alkylthio, or alkyl having 1 to 4 carbon atoms, or a cycloalkyl group having 3 to 5 carbon atoms; and R⁴ is hydrogen. To do so, the compounds of Formula I wherein R¹ is a halogen or hydrogen atom, or an alkylthio or alkyl group having 1 to 4 carbon atoms, or a cycloalkyl group with 3 to 5 carbon atoms; R² is halogen; R³ is hydrogen, halogen, or an alkylthio or alkyl group with 1 to 4 carbon atoms, or a cycloalkyl group with 3 to 5 carbon atoms; and R⁴ is acyl or hydrogen, are used as starting materials.

The introduction of alkoxy groups is also possible in accordance with the above-described method if there are alkylthio substituents present particularly in the case of those compounds wherein R¹ is halogen, R² is either alkylthio or cycloalkyl, and R³ is hydrogen, alkyl with 1 to 4 carbon atoms, or cycloalkyl, the halogen substituent can be substituted by the corresponding alkoxy group having 1 to 4 carbon atoms in the presence of alcohols or alcoholates at temperatures of between 0° C. and the boiling point of the alcohol or alcoholate used.

The conversion of halogen substituents on the pyrimidine radical to alkylthio groups is carried out in a manner analogous to that described for the substitution of alkoxy groups for the halogens. Alkylmercaptans are used and, it has been found, the preferred temperature range is from 20° C. to the boiling temperature of the solvent. The method is operable as low as O° C. As in the case of the alkoxy introduction, leaving at least one halogen atom bonded to the pyrimidine radical and available for synthesis of other compounds of this invention is useful. It permits an exchange in the presence of alkoxy substituents. The additional halogens can be reacted under more extreme conditions of temperature reaction time and alkali mercaptide concentration. The preferred material is a suspension of sodium methyl mercaptide in toluene.

In the foregoing manner, compounds having alkylthio substituents are preferably obtained from the compounds of Formula I wherein R¹ and R³ are halogen, hydrogen, or alkyl or alkoxy with 1 to 4 carbon atoms or a cycloalkyl group having 3 to 5 carbon atoms; R² is halogen, and R⁴ is hydrogen or an aliphatic acyl group. The compounds can be obtained by substituting the halogen substituents with the desired radicals.

By a suitable selection of reaction conditions, some or all of R¹, R², and R³ may be halogen atoms and such compounds can be used for further substitution by alkoxy or alkylthio groups in any desired order or combination.

The compounds for Formula I can also be prepared by reacting the *S*-alkylisothiouronium salts of Formula IV 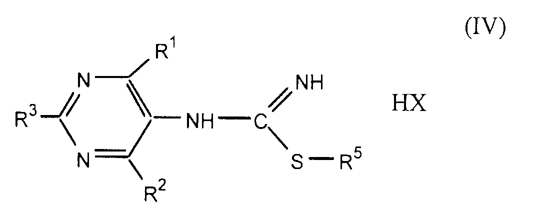 with ethylene diamine in a known manner, where R⁵ is alkyl, preferably methyl and X is halogen. One method for carrying out this reaction is by heating the two reactants for several hours in solvents, such as alcohol. Methanol is particularly preferred. In this reaction, the *S*-methylisothiouronium salts are preferred.

The starting compounds for the preparation of the compounds of Formula IV can be obtained by known methods from the corresponding 5-aminopyrimidine derivatives of Formula II 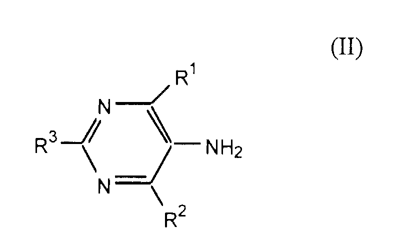 It is only necessary to start from those compounds wherein R¹, R², and R³ represent the appropriate desired substituents.

The compounds of Formula II can be reacted with benzoylisothiocyanate to form the corresponding benzoyl thioureas. These can be hydrolized to the pyrimidyl thioureas of Formula V 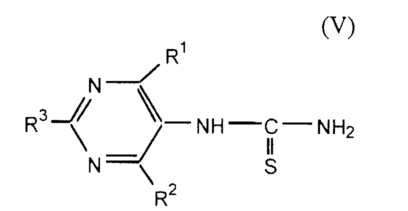 The benzoylisothiocyanate is obtained by the reaction between ammonium thiocyanate and benzoylchloride. The compounds of Formula V can then be reacted with methyliodide or other similar methylating agents to form the *S*-methylisothiouronium salts of Formula IV.

The pyrimidyl benzoylthioureas are preferably prepared by the reaction of ammonium thiocyanate, benzoylchloride and aminopyrimidine in a heated solvent, preferably boiling acetone. The thiourea derivatives obtained are then hydrolized in order to separate the benzoyl radical. The hydrolization can take place, for example, in the presence of bases and, if necessary, with heating. Lye is particularly suitable for this purpose. The substituted thioureas are suspended, for example in acetone, and converted by methylating agents (preferably methyl iodide) to isothiouroniumiodide. The reaction is preferably carried out in boiling acetone.

Moxonidine, 4-chloro-*N*- (imidazoline-2-ylidene) -6-methoxy-2-methyl-5-pyrimidineamine, is prepared generally as disclosed in U.S. Patent 4,323,570. Preferably, 4-chloro-*N*- (imidazoline-2-ylidene) -6-methoxy-2-methyl-5-pyrimidineamine is prepared as follows.

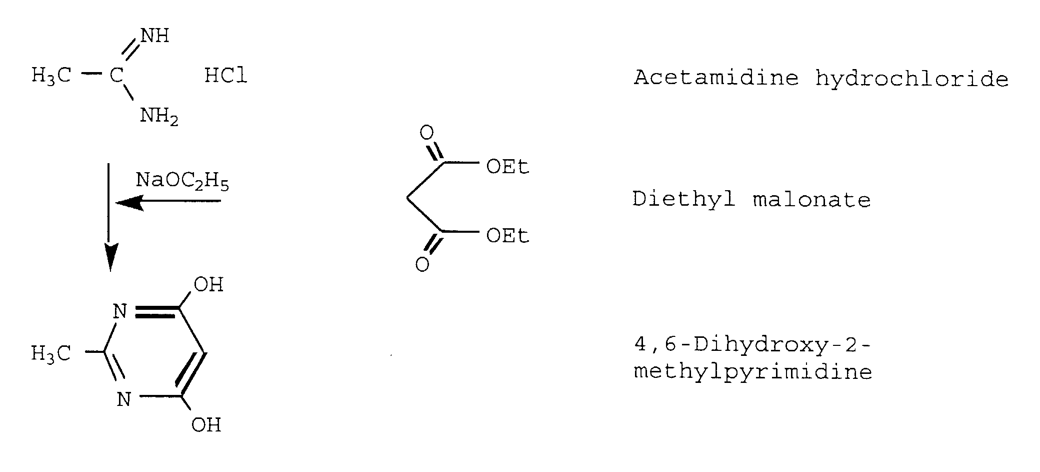

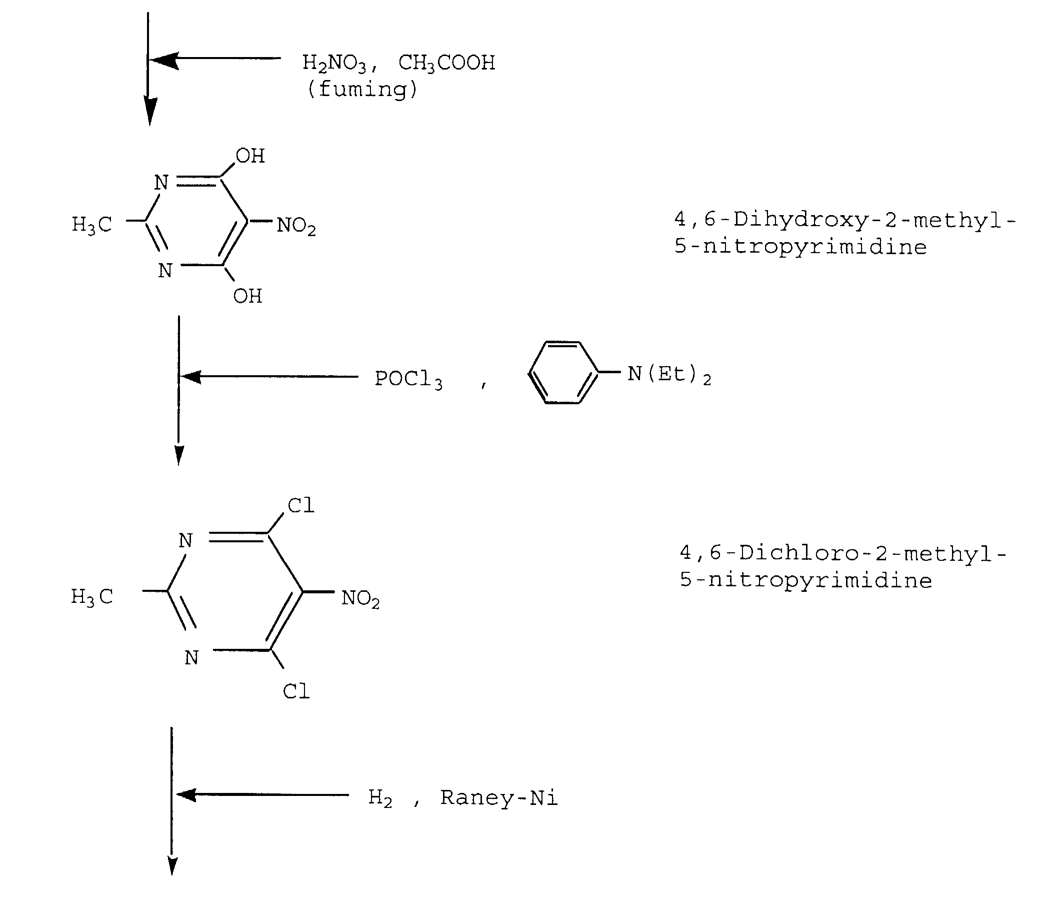

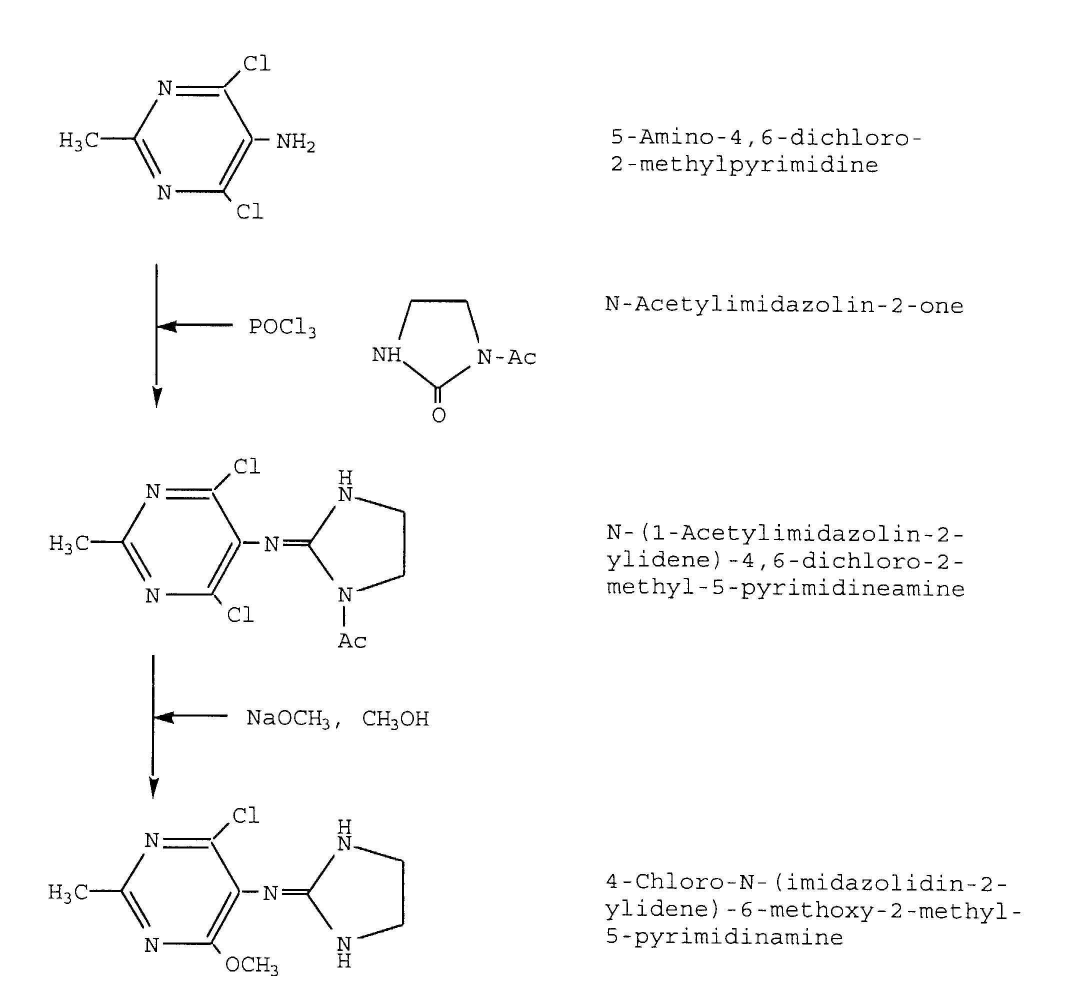

N-acetylimidazoline-2-one is prepared by reacting acetic anhydride with 2-imidazolidone at room temperature. The reaction mixture is heated to between 80 °C and 100 °C for 90 minutes and then cooled to from about 10 °C to about -10 °C to afford N-acetylimidazoline-2-one.

The first intermediate, 4,6-dihydroxy-2-methylpyrimidinamine, is synthesized by preparing sodium ethoxide in situ from sodium and anhydrous ethanol under a nitrogen blanket. Acetamidine hydrochloride and diethyl malonate are added and the reaction mixture heated to boiling for 2 to 5 hours to afford 4,6-dihydroxy-2-methylpyrimidine.

The second intermediate, 4,6-dihydroxy-2-methyl-5-nitropyrimidine, is then synthesized by slowly adding 4,6-dihydroxy-2-methylpyrimidine to a reaction mixture of fuming nitric acid in acetic acid. Once addition of 4, 6-dihydroxy-2-methylpyrimidine is complete, the reaction mixture is stirred for one-half to 2 hours to afford 4,6-dihydroxy-2-methyl-5-nitropyrimidine.

Following the nitration, phosphorous oxychloride (POCl₃) and 4,6-dihydroxy-2-methyl-5-nitropyrimidine are combined with stirring. To this mixture, diethylaniline is added dropwise at a rate so that the reaction mixture temperature is maintained below about 40 °C. After the addition is complete, the reaction mixture is refluxed for one to three hours and then distilled under a vacuum to afford the third intermediate, 4,6-dichloro-2-methyl-5-nitropyrimidine.

The third intermediate, 4,6-dichloro-2-methyl-5-nitropyrimidine is hydrogenated over Raney-Ni as a 10% to 30% solution in toluene to afford the corresponding compound, 4,6-dichloro-2-methyl-5-aminopyrimidine, as a fourth intermediate.

The fifth intermediate, N-(l-acetylimidazolin-2-ylidene) -4,6-dichloro-5-pyrimidinamine, is then prepared by combining phosphorous oxychloride, N-acetylimidazolin-2-one and 5-amino-4,6-dichloro-2-methylpyrimidine, and heating to boiling over from 2 to 4 hours, and then cooling, with stirring to room temperature.

The final product, 4-chloro-N- (imidazolin-2-ylidene) -6-methoxy-2-methyl-5-pyrimidinamine is synthesized by first preparing sodium methoxide in situ from anhydrous methanol and sodium. The fifth intermediate, N-(1-acetylimidazolin-2-ylidene) -4,6-dichloro-2-methyl-5-pyrimidinamine, is added and the reaction mixture brought to a boil. From 15 minutes to 1 hour after the reaction mixture is brought to a boil, further sodium methoxide is added and the reaction mixture is maintained at a boil for from 15 minutes to 1 hour to afford 4-chloro-N- (imidazolin-2-ylidene) -6-methoxy-2-methyl-5-pyrimidinamine.

Work-up of the several intermediates are carried out by standard techniques well-known to those skilled in the art. The various reactants and reagents used in this synthesis are commercially available or readily prepared from commercially available material by standard methods well-known to those skilled in the art.

It will be appreciated that the compounds of the present invention may be isolated per se or may be converted to an acid addition salt using conventional methods. As mentioned above, the invention includes pharmaceutically acceptable salts of the compounds of Formula I. The substituted aminopyrimidines of Formula I can react with any of a number of inorganic and organic acids, to form a pharmaceutically acceptable salt. Acids commonly employed to form acid addition salts are inorganic acids such as hydrochloric acid, hydrobromic acid, hydroiodic acid, sulfuric acid, phosphoric acid, and the like, and organic acids such as p-toluene-sulfonic, methanesulfonic acid, oxalic acid, p-bromophenyl-sulfonic acid, carbonic acid, succinic acid, citric acid, benzoic acid, acetic acid, and the like. Examples of such pharmaceutically acceptable salts thus are the sulfate pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycollate, tartrate, methanesulfonate, propanesulfonate, naphthalene-1-sulfonate, naphthalene-2-sulfonate, mandelate, and the like. Preferred pharmaceutically acceptable acid addition salts are those formed with mineral acids such as hydrochloric acid, hydrobromic acid and sulfuric acid.

In addition, the substituted aminopyrimidines of Formula I, or their pharmaceutically acceptable salts may form solvates with water or common organic solvents such as ethanol. These solvates are also contemplated as useful in the methods of this invention.

The acid addition salts are typically formed by reacting a compound of Formula I with an equimolar or excess amount of acid. The reactants are generally combined in a mutual solvent. The salt normally precipitates out of solution within about one hour to 10 days and can be isolated by filtration or the solvent can be stripped off by conventional means.

The pharmaceutically acceptable salts generally have enhanced solubility characteristics compared to the compound from which they are derived, and thus are often more amenable to formulation.

As noted above, the compounds of Formula I, particularly moxonidine, and the pharmaceutically acceptable salts and solvates thereof, are useful for inhibiting glucose metabolism deterioration in mammals having glucose metabolism abnormalities and at risk for developing glucose metabolism abnormalities, prior to the onset of hyperglycemia, thereby preventing, delaying or remitting glucose metabolism deterioration in the presence of a glucose metabolism abnormality etiology. Non-limiting examples of such glucose metabolism abnormality etiologies include, impaired glucose tolerance, insulin resistance, circulating islet-cell antibodies, hyperinsulinemia, hypoinsulinemia (in the presence of functioning beta cells), increased hepatic glucose output, and obesity.

The term "impaired glucose tolerance" means individuals whose plasma glucose concentrations lie between normal and values diagnostic of frank diabetes. Plasma glucose concentrations in such individuals may progress to overt diabetes over variable time periods.

Impaired glucose tolerance can be determined by an oral glucose tolerance test. The World Health Organization classification defines impaired glucose tolerance with a two-hour plasma glucose concentration between 140 and 200 mg/dl (7.8 to 11.1 mmol/L) after a standard glucose challenge in persons with a fasting glucose concentration of less than 140 mg/dl (7.8 mmol/L). The National Diabetes Data Group and World Health Organization classifications differ in that nondiagnostic or unclassifiable oral glucose tolerance test values occur in the National Diabetes Data Group system but not in that of the World Health Organization. Under standardized conditions of testing, the two-hour value alone may be adequate for diagnosis.

"Glucose metabolism abnormality" means one or more conditions or disorders that cause, or tend to cause, or if left uncompensated will cause blood glucose levels to be greater than normal. Generally, such blood glucose levels are a result of insulin deficiency or resistance of the body's cells to the action of insulin. Blood glucose levels in the range that causes a clinical diagnosis of diabetes are frankly hyperglycemic, while values above normal, and below frankly hyperglycemic are indeterminate.

The term "at risk for developing glucose metabolism abnormalities" means individuals with a previous abnormality of glucose metabolism, and individuals with a potential abnormality of glucose metabolism.

The term "previous abnormality of glucose metabolism" means individuals with previous diabetes or impaired glucose metabolism who regained normal glucose metabolism. Most frequently, individuals in this class include patients with former gestational diabetes, impaired glucose tolerance, or Type II diabetes whose glucose metabolic status has returned to normal. These individuals are at increased risk for developing diabetes.

The term "potential abnormality of glucose metabolism" means individuals at substantially greater risk for developing a glucose metabolism abnormality and/or diabetes than the general population. The category includes first-degree relatives of patients with diabetes, an identical co-twin of a patient with Type I or Type II diabetes, human-lymphocyte-antigen-identical sibling, or an individual with circulating antibodies directed against the islet-cell (ICA). Patients in this latter group may demonstrate islet-cell antibodies in sera for some years before progressing toward overt diabetes. Also included are obese individuals and individuals who have delivered a large infant (nine pounds or greater).

As described above, insulin-dependent (Type I) diabetes mellitus is characterized by complete or nearly complete insulin deficiency, and the need for insulin replacement therapy. The metabolic disturbances (abnormalities) that characterize non-insulin-dependent (Type II) diabetes mellitus occur less acutely than those in Type I disease, and insulin replacement generally is not needed in the early stages. Initially, diet therapy alone may be sufficient. Later, oral agents or insulin may be required.

The central role of insulin resistance in the pathogenesis of Type II diabetes mellitus is widely accepted. Insulin resistance is defined as the subnormal biologic response to a given concentration of insulin. Given the pleiotropic actions of insulin on glucose, lipid, and protein metabolism, multiple defects could result in hyperinsulinemia or insulin resistance. Insulinemia, especially levels of fasting insulin, correlate surprisingly well (at the level of 0.6-0.8) with more sophisticated measures of insulin resistance such as the euglycemic clamp or the minimal model.

Individuals who develop impaired glucose tolerance from normal glucose tolerance demonstrate that insulin resistance preceeds a defect in insulin secretion. Recent studies suggest a two-step process, the first being insulin resistance and the second being β-cell failure. Loss of pulsatile insulin secretion has also been suggested as the first lesion leading to Type II diabetes mellitus.

Chronic hyperglycemia is believed detrimental to insulin secretion and may also induce insulin resistance. This gluco-toxicity may perpetuate the diabetic state and eventually lead to a permanent loss of β-cell function.

Elevated urinary albumin excretion occurs early in the course of Type II diabetes mellitus, in persons with impaired glucose tolerance, and in nondiabetic offspring of Type II diabetes mellitus subjects.

Impaired glucose tolerance (IGT) is defined as a glycemic response to the standard 75-g oral glucose challenge that is intermediate between the normal and diabetic criteria established by the U.S. National Diabetes Data Group, i.e., venous plasma or capillary whole blood glucose concentration 140-199 mg/dl at 2 hours after a glucose load. The importance of impaired glucose tolerance as a prognostic category for development of Type II diabetes is generally accepted. Those patients in this group have higher fasting blood glucose levels, that is, between 125 and 140 mg/dl have the greatest risk of developing Type II diabetes mellitus.

Genetic susceptibility to Type II diabetes is likely to include separate but interrelated genes for insulin resistance, obesity, fat distribution, and other specific gene defects. Some of these have been identified, others are still unknown. Major behavioral factors include dietary calories and fat, physical inactivity, and weight gain. Cultural and psychosocial factors may operate through diet, activity, and weight gain, and may also operate independently. It appears that these behavioral factors can operate not only to start the cascade but also as promoters during progression to Type II diabetes mellitus.

Weight gain is the major mechanism etiologically associated with insulin resistance. Insulin resistance may be a feedback mechanism to prevent further weight gain above that determined by some set point. Rapid weight gain increases insulin resistance in subjects with hyperandrogenism, insulin resistance, and acanthosis nigricans. On the other hand, weight loss in severely obese subjects appears to deter progression from IGT to Type II diabetes mellitus.

Insulin responses increase during the progression of glucose intolerance from normal to impaired. However, there is a progressive fall in insulin secretion as glucose levels rise, either by unmasking a primary defect in insulin secretion, from "pancreatic exhaustion," from glucose toxicity, or from a combination of these defects. Prospective studies of persons with IGT have identified a decrease in insulin levels after a glucose challenge (consistent with a secretory defect) as a predictor of the development of Type II diabetes mellitus.

Unlike Type I diabetes, in which glucose metabolism abnormalities are thought to result primarily from absolute insulin deficiency caused by loss of β-cell mass, Type II diabetes is recognized as a heterogeneous disorder having one or more of three defects (glucose metabolism abnormalities). These include abnormalities in insulin secretion, reduced sensitivity to insulin (peripheral resistance), and excessive hepatic glucose production in the fasted state (although this third category may be a consequence of the first two).

The failure of many patients with mild-to-moderate hyperglycemia to develop ketoacidosis has long indicated the presence of endogenous insulin. Investigations have focused on four aspects of insulin secretion: 1) Fasted serum insulin concentrations; 2) Serum immunoreactive insulin profiles immediately after an intravenous glucose challenge, called the early-phase response; 3) Insulin secretion patterns after the early phase, called the late-phase response; and 4) Sum of the insulin secretion from the two phases, or total insulin secretion

Characteristically, the early phase of insulin secretion following administration of glucose or food is decreased or absent in patients and can be considered a "marker" for early Type II diabetes. In some cases, therapeutic measures such as weight reduction and physical exercise may improve Type II diabetes, however, the abnormalities in early-phase insulin secretion remains.

Insulin secretory defects also include aberrations in the pulsatile nature of secretion; and subnormal, normal, or elevated serum total insulin responses, which are insufficient to reduce ambient hyperglycemia. Also, in most obese (nondiabetic or diabetic) individuals, basal and stimulated insulin concentrations (and secretion rates) are elevated. This suggests that both a defect in the β-cell responsiveness to the glucose signal and a peripheral insensitivity or resistance to insulin contribute to the pathogenesis of Type II diabetes.

Insulin resistance exists when a normal serum insulin concentration produces a subnormal response or when elevated insulin is required to produce a normal response. Peripheral insulin resistance accounts for most increases in postprandial blood glucose concentrations since 70 to 90% of ingested glucose is disposed of in the periphery. Causes of insulin resistance include obesity; diabetes due to antibodies to the insulin receptor and other conditions related to receptor dysfunction; abnormal "insulin" secreted by the pancreas; hyperproinsulinemia due to a failure of cleavage of the connecting peptide from proinsulin; mutant insulin receptor as a result of aberration in the insulin receptor gene; humoral insulin antagonists; physiologic and endocrinopathic hypersecretion of the counterregulatory hormones-glucagon, epinephrine, cortisol, and growth hormone; immunologic insulin antagonists; antibodies to animal insulins and miscellaneous; free fatty acids.

All of the conditions which cause insulin resistance are characterized by single or combined defects that occur before, during, or after the insulin-receptor interaction (prereceptor, receptor, or postreceptor stage).

Patients with Type II diabetes showed reduced glucose disposal rates, demonstrating a tissue defect in the handling of glucose. Adipocytes from persons with impaired glucose tolerance or Type II diabetes bind less insulin than adipocytes from normal subjects. Diminished insulin binding is due to a decrease in the number of insulin receptors rather than to lessened binding affinity. The principal tissue responsible for peripheral insulin insensitivity is not fat but skeletal muscle. Decreased insulin binding in obesity results from a reduced number of binding sites. Deficient cellular or tissue responses to insulin contribute to hyperglycemia.

In patients with Type II diabetes, hepatic glucose output is approximately 0.5 mg/kg/min greater than normal, corresponding to a difference of 50 g of glucose after 24 hours in a 70-kg person. The liver of a normal individual produces glucose at a rate of approximately 2.2 mg/kg/min. A number of studies have indicated a close correlation between hepatic glucose output and fasting hyperglycemia in patients with Type II diabetes. Since moderate fasting hyperinsulinemia (usually in the range of 20 to 50 µU/ml) is frequently found in Type II diabetes, its failure to reduce hepatic glucose output indicates the presence of hepatic insulin resistance. Hepatic glucose output is more than 90% suppressed in patients with Type II disease when serum insulin concentrations are elevated to 100 µU/ml or more.

While the biochemistry of increased glucose flux from the liver is not well understood, certain data and hypotheses are worthy of mention. First, elevated glucagon concentrations are consistently found in Type II diabetes. It has been suggested that the failed suppression of hyperglucagonemia and the consequent increased hepatic glucose output in Type II diabetes could be related to the loss of the normal pulsatile bursts of insulin.

Further, increased conversion of glucose to lactate by the glycolytic (Cori) cycle could occur in skeletal muscle because glucose is not converted to glycogen in skeletal muscle, thus resulting in recycling of the 3-carbon substrate to the liver. In the liver, lactate is converted to glucose then recycled to the muscle, where it is reconverted to 3-carbon fragments, then recirculated and so on. Energy to sustain this sequence of events is supplied by oxidation of fatty acids in the liver and could explain the increased free-fatty-acid concentrations associated with hyperglycemia in Type II diabetes. Specifically, in the Hales-Randle cycle, free fatty acids are taken up and oxidized within the muscle cells. Increased free-fatty-acid oxidation inhibits glycolysis and glucose uptake, actions which oppose the effect of insulin action.

Excessive hepatic glucose output is the most important contributor to the basal hyperglycemia observed in Type II diabetes. Patients with impaired glucose tolerance have either no or subtle increase in hepatic glucose output since they have normal fasting blood glucose concentrations. In these patients, the degree of insulin deficiency reduces glucose uptake in muscle.

Hyperglycemia resulting from the interaction of impaired insulin secretion, peripheral resistance to insulin, and increased hepatic glucose output may be toxic to the β-cell and result in further worsening of diabetes.

In addition to Type I and Type II, a subclass termed Other Types of diabetes mellitus is recognized. This Other Types subclass is heterogeneous and includes specific disorders such as pancreatic disease, hyperendocrinopathies, chemical-agent - or drug-induced diabetes, or genetic syndromes associated with glucose intolerance, each of which comprises a glucose metabolism abnormality.

In clinical practice, the suspicion of a glucose metabolism abnormality and those at risk for developing a glucose metabolism abnormality is gleaned from individual medical history and physical findings. Symptoms such as fatigue, thirst, polyuria, weight loss, and recurrent infection are frequent clues. A family history of diabetes, obesity, unfavorable obstetrical experiences, premature atherosclerosis, or neuropathic disorders are indicators. Urine glucose testing is frequently performed as a screening test, but is not acceptable for diagnostic purposes. A positive urine test is usually a helpful indicator, but may give misleading results during pregnancy because of increased renal blood flow or in the presence of renal glycosuria. Patients with high renal glucose thresholds or elderly patients with decreased renal blood flow may not have glucosuria in spite of elevated blood glucose concentrations.

Diagnostic criteria for diabetes are used for ascertaining a glucose metabolism abnormality and individuals at risk for developing a glucose metabolism abnormality. These diagnostic criteria are based on venous plasma glucose values using glucose analytical methods. Plasma (or serum) is preferred over whole blood for glucose determinations because there is less interference with equipment, differences in hematocrit are eliminated, and many nonglucose-reducing substances are removed. Capillary blood, which provides values similar to arterial whole blood glucose concentrations, approximates venous plasma. Automated equipment is used to process venous plasma for determination of glucose concentration with either ferricyanide, hexokinase, glucose oxidase, or orthotoluidine reagents. To interpret laboratory test results correctly, clinicians must have information concerning the methods used in their referral laboratory and be aware of the laboratory's normal range of values.

In normal subjects, the currently generally accepted upper normal limit of fasting plasma glucose is 115 mg/dl (6.4 mmol/L). Diabetes can be diagnosed reliably when fasting plasma glucose concentrations are greater than or equal to 140 mg/dl (7.8 mmol/L) (i.e. hyperglycemic). An aid in diagnosis is to determine the glycosylated hemoglobin concentration. If the glycosylated hemoglobin concentration is greater than normal, the diagnosis must be confirmed by an elevated ambient glucose concentration preceding the fasting plasma glucose determination; otherwise, tests for an elevated fasting plasma glucose should be done on two occasions to eliminate the possibility of error. The advantages of measuring the fasting plasma glucose concentration to test for diabetes are that age, previous caloric intake, or activity does not affect plasma glucose and conditions for obtaining a proper blood sample are standardized. With few exceptions, fasting plasma glucose values correlate well with the rise in plasma glucose concentrations observed after a meal or glucose challenge.

A two-hour post-oral glucose load plasma glucose concentration of greater than or equal to 200 mg/dl (11.1 mmol/L) may be indicative of diabetes if certain precautions have been observed. Because glucose utilization is impaired in persons consuming low-carbohydrate or weight-reduction diets, the post-oral glucose load glucose test should be performed in those consuming unrestricted diets providing greater than 150 g of carbohydrate daily for at least three days. Additional precautionary measures include eliminating drugs that reduce glucose tolerance, having individuals maintain normal physical activity, and avoiding the test in ill or stressed persons; a specific test meal has not been adequately validated for diagnostic screening. The test is most reliably performed using a standard oral glucose load. In patients with liver or kidney disease, partial gastrectomy, or thyrotoxicosis, an elevated postprandial plasma glucose may be observed in the absence of diabetes. Conversely, in an obviously healthy patient with a family history of diabetes, the combination of a normal fasting plasma glucose and an abnormal elevation of postglucose values (greater than or equal to 200 mg/dl; 11.1 mmol/L) may be indicative of glucose metabolism abnormalities. Subsequent testing, corroborated by glycohemoglobin determinations, should be performed to verify the diagnosis in this situation.

The oral glucose tolerance test is the most sensitive test for the diagnosis of diabetes. Without scrupulous care in standardizing the test and preparing the patient, however, the oral glucose tolerance test can be misleading since many factors can interfere with normal glucose tolerance and result in a hyperglycemic curve. This test is not required for diagnostic purposes when the fasting plasma glucose is unequivocally elevated, or if blood sugars are repeatedly greater than 200 mg/dl in the presence of hyperglycemic symptoms.

Before the oral glucose tolerance test, it is recommended that the patient receive a balanced diet providing carbohydrate intake greater than 150 g daily for a minimum of three days while maintaining normal daily activities. Carbohydrate restriction or bed rest impairs glucose tolerance. Acute illness; stress; or the use of drugs such as 5,5-diphenyl-2,4-imidazolidinedione, anovulatory agents, diuretics, and glucocorticoids can adversely influence the results.

The oral glucose tolerance test is performed in the morning after a 10- to 16-hour fast. A glucose load of 75 g is given in 300 ml of flavored beverage and consumed within five minutes after the fasting blood sample is drawn. The patient remains at rest and does not smoke or consume fluids, especially caffeine, during the procedure. Blood samples are drawn at 30-minute intervals for two hours using tubes containing sodium fluoride (30 mg/5 ml blood). Plasma is separated by centrifugation within four hours and frozen if the glucose analysis is not performed promptly.

The oral glucose tolerance test adopted by the World Health Organization requires only two plasma glucose values: fasting and two hours after a 75-g glucose load. The intervening samples at 0.5, 1, and 1.5 hours are not used. The scheme provides a simple and accurate method for obtaining diagnostic criteria. Both sets of diagnostic criteria specify that a fasting plasma glucose concentration greater than or equal to 140 mg/dl (7.8 mmol/L) and a two-hour plasma glucose concentration greater than or equal to 200 mg/dl (11.1 mmol/L) are indicative of diabetes mellitus in the nonpregnant adult. The latter value represents the concentration observed in the bimodal distribution of two-hour plasma glucose concentrations in epidemiologic studies, and is the glucose concentration at which characteristic microvascular lesions such as diabetic retinopathy appear.

The diagnosis of impaired glucose tolerance is based on the oral glucose tolerance test after the fasting plasma glucose concentration has been determined to be less than 140 mg/dl (7.8 mmol/L), as in the criteria of the U.S. National Diabetes Data Group and the World Health Organization. Individuals having a fasting plasma glucose concentration between 115 and 139 mg/dl (6.4-7.8 mmol/L) or from 140 to 200 mg/dl (7.8 to 11.1 mmol/L) at two hours are classified as having impaired glucose tolerance.

Gestational diabetes is diagnosed using criteria established by O'Sullivan and Mahan, "Criteria For the Oral Glucose Tolerance Test in Pregnancy," Diabetes, 13, 278-285 (1964). Glycosylated hemoglobin determinations are not useful in the detection of this condition. The values for fasting plasma glucose and plasma glucose concentrations are lower in the gestational diabetes criteria than in those for diabetes mellitus in the nonpregnant adult because of ties to fetal risks. Modifications of the glucose tolerance test given during pregnancy conform to their recommendations. With the same precautions, the patient receives 100 g of glucose, and blood samples are drawn at one-hour intervals for three hours after administration of the glucose load.

The World Health Organization Expert Committee has recommended that the diagnostic procedures and criteria for pregnant women remain the same as those for all adults.

Screening for gestational diabetes is recommended at the outset of pregnancy for those at risk for developing diabetes and everyone should be tested at 24 to 28 weeks. A practical screening procedure for plasma glucose measurement is to administer a 50-g glucose load and obtain a blood sample at one hour in the absence of fasting. A test is positive if the plasma glucose concentration is greater than or equal to 150 mg/dl (8.3 mmol/L). - If the screening test is positive, the oral glucose tolerance test is performed using the preparation and precautions described in Freinkel et al., Diabetes Care, 3, 499-501 (1980).

Classic symptoms of diabetes are present in 95% of children with diabetes. Since their random plasma glucose concentration exceeds 200 mg/dl (11.1 mmol/L), the oral glucose tolerance test is seldom required for diagnosis. When the oral glucose tolerance test is required, it is performed under the same standardized procedures as those for adults, except the glucose load is modified to 1.75 g/kg of ideal body weight up to 75 g. The recommended diagnostic criteria are the same as those for adult patients, except that both fasting (greater than 140 mg/dl; 7.8 mmol/L) and postload plasma glucose concentrations (greater than 200 mg/dl; 11.1 mmol/L) must be elevated for a definitive diagnosis of diabetes. Impaired glucose tolerance in children is diagnosed if the fasting plasma glucose concentration is less than 140 mg/dl (7.8 mmol/L) and the two-hour value is greater than 140 mg/dl (7.8 mmol/L) even if one of the postload values exceeds 200 mg/dl (11.1 mmol/L).

Further details of the diagnostic criteria and test procedures are set forth in the National Diabetes Data Group: Classification and diagnosis of diabetes mellitus and other categories of glucose intolerance. Diabetes 1979; 28: 1039-1057 and World Health Organization Expert Committee: Second report on diabetes mellitus. Technical report series No. 646. Geneva, Switzerland, 1980.

The rapid intravenous glucose tolerance test has been used primarily as an investigative tool and generally is not recommended for clinical use. This procedure has the advantage of greater reproducibility than the oral glucose tolerance test. (For details, see Soeldner JS: The intravenous glucose tolerance test, in Fajans SS, Sussman KE [eds]: *Diabetes Mellitus: Diagnosis and Treatment.* New York, American Diabetes Association Inc., 1971, vol. 3, pp. 107-110). The intravenous glucose tolerance test also may be used for patients unable to complete an oral glucose tolerance test because of vomiting and for those in whom the oral test is not interpretable.

The connecting peptide (C-peptide) radioimmunoassay is useful in certain conditions when information is needed regarding endogenous insulin secretion, Recent Advances in Obesity and Diabetes Research, Raven Press, New York, 1984, pp. 139-150. The C-peptide is cleaved from the pro-insulin molecule and is packaged with insulin in secretory granules so that both are released during the insulin-secretion process. Insulin antibodies in the blood of patients treated with exogenous insulin preclude the use of radioimmunoassay for the circulating insulin hormone. Partial suppression of insulin secretion by administration of exogenous insulin can be shown by C-peptide radioimmunoassay. Using C-peptide determinations, two cohorts of Type I diabetes have been described: stable insulin-dependent patients with C-peptide response to glucose or glucagon stimulation, and labile patients in whom C-peptide is absent. In Type II diabetes, C-peptide may be normal or reduced, depending on insulin secretory responses which may change during the course of treatment. Randel, J. Clin. Endocrinol. Metab., 57, 1198-1206 (1983). C-peptide levels are low or absent in patients with factitious hypoglycemia due to surreptitious insulin administration.

Glycosylated hemoglobin determination is not recommended as a criterion for the diagnosis of diabetes because of a lack of glycohemoglobin standards, difficulty in quality control, and overlapping values for patients with diabetes, and nondiabetic individuals. It may be used, however, to confirm the diagnosis. Currently, glycosylated hemoglobin concentrations are measured in patients with Type I diabetes primarily to assess glycemic control over periods of 6 to 12 weeks. Glycosylated hemoglobin (HA_{1C}) is clearly tied to complications in Type I diabetes. N. Eng. J. Med. 329 (14) 977-986 (1993).

In severe stress, such as acute illness or injury, vascular catastrophe, or major surgery, a marked rise in catecholamine concentrations occurs, following by stimulation of adrenocorticol hormone secretion. These counterregulatory hormones, together with growth hormone and glucagon, reduce the tissue sensitivity to insulin and increase hepatic glucose production while epinephrine decreases insulin secretory release. The consequence is abnormal elevation of glucose concentrations in the fasting a well as in the fed state. This condition may persist for several weeks, as demonstrated in postmyocardial infarction studies. Testing for diabetes will yield unreliable results until recovery from the stressful event has occurred.

Liver disease is associated with an abnormal elevation of blood glucose values after a glucose challenge. The liver is also associated with hyperglycemia and to failure of glycogen storage, an important source of glucose during fasting. The finding of a normal fasting plasma glucose concentration in patients with liver disease in whom the postglucose values are abnormal has been termed hepatic dysfunctional hyperglycemia. In reversible liver disorders, such as hepatitis, the glucose tolerance returns to normal upon recovery from hepatic impairment. Diabetes mellitus is diagnosed in the presence of chronic liver disease when the fasting plasma glucose value is abnormally elevated.

Renal disease also may be associated with impaired glucose tolerance, a condition termed "pseudodiabetes or uremia." Several factors contribute to this abnormality; the most significant appears to be a decrease in tissue sensitivity to insulin. Severe potassium depletion seen in chronic renal disease, despite normal serum levels, also has been cited as a cause of impaired glucose tolerance in uremia and clearly causes failure of insulin secretion. Diabetes mellitus is diagnosed in these patients when the fasting plasma glucose concentration is elevated or, in some situations, when diabetic retinopathy is present and nodular glomerulosclerosis is seen on renal biopsy.

A number of commonly used drugs may induce diabetes, among them diuretics used frequently in the management of hypertension. Prospective studies have shown a 7% greater prevalence of diabetes in patients receiving thiazide than in hypertensive patients treated without these agents. Diabetic ketoacidosis and nonketotic hyperosmolar coma have been observed in patients receiving diuretics. Although the underlying mechanism of this form of diabetes is unclear, the leading consideration is depletion of intracellular potassium (not always reflected in serum values). Administration of potassium may ameliorate glucose intolerance induced by thiazides although thiazides inhibit insulin release even when potassium is taken into account. In patients with known diabetes, the impairment of carbohydrate intolerance is improved by increasing the dosage of drug or other diabetes care agent. Other conditions associated with potasium depletion, such as hyperaldosteronism, have been marked by glucose intolerance, which is improved by potassium administration.

Another form of drug-induced disease is glucocorticoid-induced diabetes, which occurs in 14% of patients who receive glucocorticoid therapy as treatment for a variety of diseases. This adverse effect is usually observed with prolonged oral use of these agents, and also has been noted with extensive topical use of steroids in dermatologic disorders. The factors involved in glucose intolerance are thought to involve a reduced affinity of insulin for its tissue receptor where insulin action is initiated, together with an increase in hepatic glucose production through stimulation of gluconeogenesis. This form of diabetes is infrequently associated with ketonuria and acidosis. Cessation of steroid therapy is usually accompanied by improvement in glucose tolerance, but occasionally insulin requiring diabetes persists. The latter may occur in patients in whom undetected diabetes was unmasked by steroid therapy. The oral contraceptive steroids may exhibit a mild diabetogenic effect, although this influence appears to be no significant diabetogenic effects associated with other contraceptive preparations.

A rare but important form of diabetes, termed insulin resistance with acanthosis nigricans, has been described in which alterations in the function of the insulin receptor is the predominant abnormality. Two types of disorders are included in this category. Type A is usually observed in young women with features of virilization, polycystic ovaries, and hyperandrogenism. In these patients, reduced insulin-receptor activity or postreceptor defects with normal insulin binding result in impaired insulin action and insulin resistance. Type B insulin resistance is caused by the production of antibodies to the insulin receptor. The insulin-receptor antibody, which blocks access of insulin to the insulin receptor, results from an autoimmune-type response seen in patients with systemic lupus erythematosus or other autoimmune states. In early stages of this disorder, or in tissue cultures, this antibody may function as an insulin agonist, in which its coupling with the receptor may produce insulin effects resulting in severe or even fatal hypoglycemia. A frequent effect, however, is intense hyperglycemia and insulin resistance resulting from the interference of insulin binding to tissue receptors. Diagnosis of this condition can be established by the demonstration of insulin-receptor antibody in the patient's serum.

Nearly one-third of patient with diabetes are over 60 years of age. The increasing prevalence of diabetes may be related, in part, to the increasing longevity of the population. Early studies have demonstrated a gradual decrease in glucose tolerance with advancing age. The basis for impairment of postglucose curves has been shown to be related to the reduced tissue sensitivity to insulin rather than to a decrease in insulin availability. These data, however, are not applicable to the diagnosis of diabetes in the elderly. The current diagnostic criteria of the National Diabetes Data Group and the World Health Organization apply to all age groups and are higher than values for the age-related gradients previously used in the interpretation of the oral glucose tolerance test in the elderly.

Among the elderly, the classic symptoms of diabetes are not always manifest. Clinical presentations are often those related to complications of the disease, such as vascular disorders or neuropathic syndromes. To arrive at the correct diagnosis, physicians must be alert to the clinical signs of diabetes that may appear in various organ systems of the elderly. Early diagnosis is needed to apply appropriate treatment and therefore to forestall hyperglycemia and the progression of diabetic complications and improve the general health, well being, and vigor of these patients.

Recommended for diagnosis of diabetes in the elderly is a fasting plasma glucose concentration greater than or equal to 140 mg/dl (7.8 mmol/L). The sedentary or infirm elderly patient whose dietary intake is often faulty is not a suitable candidate for glucose tolerance testing. Treatment for these patients should be conservative if there is a risk of hypoglycemia, with standards for glycemic control raised by 30 or 40 mg/dl (1.7 or 2.2 mmol/L) above those for the middle-aged, nonpregnant adult diabetic population.

Type II diabetes differs from Type I diabetes not only in its genetic features-high concordance rate in identical twins and absence of association with human leukocyte antigens (HLA) types-but in other important respects. The most striking is that β-cell destruction does not occur acutely in Type II diabetes; indeed, the pancreatic islets can be microscopically indistinguishable from those of normal subjects and contain normal or near-normal amounts of insulin. Insulin is present in the circulation and, in the nonobese, the insulin response to a glucose load is preserved, although the peak concentration may be delayed. Early Type II diabetes does not seem to result from insulin deficiency but from sluggish responsiveness, perhaps the result of a defect in the secretory control of insulin and from resistance to its action. This defect, whatever its cause, is strongly influenced by genetic factors.

Presence of diabetes in a family member is an established risk factor for diabetes, particularly Type II. In addition to genetics, diet, such as high-fat, low-carbohydrate intake, physical inactivity, obesity, and low birth weight are all generally recognized as risk factors for diabetes.

By the term "effective dose" is meant an amount of a substituted aminopyrimidine of Formula I, preferably 4-chloro-N- (imidazoline-2-ylidene) -6-methoxy-2-methyl-5-pyrimidineamine, or a pharmaceutically acceptable salt or solvate thereof, sufficient to afford the desired therapeutic or prophylactic effect. The term "treating" as used herein includes therapeutically or prophylactically administering a substituted aminopyrimidine of Formula I, preferably moxonidine to a mammal to prevent or inhibit the onset of a glucose metabolism abnormality or inhibit glucose metabolism deterioration once the condition is established, and prior to the onset of hyperglycemia. Also contemplated as within the scope of the present invention is concomitant treatment with diabetes care agents including oral hypoglycemic agents, insulin or insulin derivatives, such as lispro. The need and dosage of such diabetes care agents would be readily determined by the attending physician as circumstances dictate.

The compounds of the present invention are putative I₁-imidazoline ligands demonstrating substantial selectivity for I₁ receptors over α₂ adrenergic receptors. In saturation binding experiments in bovine rostral ventrolateral medulla (bovine RVLM), moxonidine demonstrates a selectivity value (Kᵢ at α₂ sites in µM/Kᵢ sites in µM) of greater than 20 and preferably greater than 30 X, where Kᵢ is the inhibitory affinity constant. Of course, Kᵢ is inversely proportional to affinity, so lower Kᵢ values indicate higher affinity. Thus, the higher the selectivity value, the more selective the compound. In contract, clonidine's selectivity value in bovine RVLM is less than 4. See Ernsberger et al., J. Pharmacol. Exp. Ther., 264, 172-182 (1993) for details on experimental protocol and results.

As used herein, the term "mammal" means the Mammalia class of higher vertebrates. The term "mammal" includes, but is not limited to, a human. The dose of compound to be administered, in general, is from about 0.01 to about 15.0 mg/day; as usual, the daily dose may be administered in a single bolus, or in divided doses, depending on the judgment of the physician in charge of the case. A more preferred range of doses is from about 0.02 to about 10.0 mg/day; other dosage ranges which may be preferred in certain circumstances are from about 0.05 to about 5.0 mg/day; from about 0.1 to about 2.0 mg/day; from about 0.05 to about 1.0 mg/day; and a particularly preferred range is from about 0.05 to about 1.5 mg/day. It will be understood that the dose for a given patient is always to be set by the judgment of the attending physician, and that the dose is subject to modification based on the size of the patient, the lean or fat nature of the patient, the characteristics of the particular compound (freebase or salt) chosen, the severity of the patient's symptoms and psychological factors which may affect the patient's physiological responses, as well as concomitant treatment with other diabetes care agents.

Pharmaceuticals are substantially always formulated into pharmaceutical dosage forms, in order to provide an easily controllable dosage of the drug, and to give the patient an elegant and easily handled product. The present compounds are susceptible to formulation into the conventional pharmaceutical dosage forms, including capsules, tablets, inhalants, injectable parenteral solutions and suppositories. Usually the oral dosage forms, particularly tablets and capsules, are most convenient for the patient and are usually preferred. Liquid suspensions of pharmaceuticals, formerly popular, have now become less popular and are seldom used but the present compounds are entirely amenable to such products should they be desired.

While it is possible to administer a compound of Formula I, preferably 4-chloro-*N*- (imidazoline-2-ylidene) -6-methoxy-2-methyl-5-pyrimidineamine directly, it is preferably employed in the form of a pharmaceutical formulation comprising a pharmaceutically acceptable carrier, diluent or excipient and the compound or a pharmaceutically acceptable salt or solvate thereof. Such formulations will contain from about 0.01 percent to about 99.9 percent by weight of the compound.

In making the formulations of the present invention, the active ingredient will usually be mixed with at least one carrier, or diluted by at least one carrier, or enclosed within a carrier which may be in the form of a capsule, sachet, paper or other container using conventional techniques and procedures for the preparing of pharmaceutical formulations. When the carrier serves as a diluent, it may be a solid, semi-solid or liquid material which acts as a vehicle, excipient or medium for the active ingredient. Thus, the formulations can be in the form of tablets, granules, pills, powders, lozenges, sachets, cachets, emulsions, solutions, syrups, suspensions and soft and hard gelatin capsules.

Examples of suitable carriers, diluents and excipients include lactose, dextrose, sucrose, sorbitol, mannitol, starches, gum acacia, calcium phosphate, alginates, liquid paraffin, calcium silicate, microcrystalline cellulose, polyvinylpyrrolidone, cellulose, tragcanth, gelatin, syrup, methylcellulose, methyl- and propyl-hydroxybenzoates, vegetable oils, such as olive oil, injectable organic esters such as ethyl oleate, talc, magnesium stearate, water and mineral oil. The formulations may also include wetting agents, lubricating, emulsifying and suspending agents, preserving agents, sweetening agents, perfuming agents, stabilizing agents or flavoring agents.

The compounds of the present invention can be formulated in unit dosage formulations comprising a dose between about 0.01 mg and about 15 mg. Preferably the compound is in the form of a pharmaceutically acceptable salt.

The compounds of Formula I can be administered by a variety of routes including oral, rectal, transdermal, subcutaneous, intravenous, intramuscular, and intranasal. The compound is preferably formulated prior to administration as a pharmaceutical formulation comprising an effective amount of a compound of Formula I preferably moxonidine, or a pharmaceutically acceptable salt or solvate thereof in association with a pharmaceutically acceptable carrier, diluent or excipient therefor.

By "pharmaceutically acceptable" it is meant the carrier, diluent or excipient must be compatible with the other ingredients of the formulation and not deleterious to the recipient thereof.

The present pharmaceutical formulations are prepared by known procedures using well-known and readily available ingredients. The compositions of this invention may be formulated so as to provide quick, sustained, or delayed release of the active ingredient after administration to the patient by employing procedures well-known in the art. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), soft and hard gelatin capsules, suppositories, sterile injectable solutions, sterile packaged powders, and the like.

The activity of the compounds of the present invention was determined by testing the efficacy in vivo in Zucker diabetic rats.

The Zucker diabetic fatty rat (ZDF-DRT-fa) is a classic rodent model of diabetes associated with obesity and insulin resistance, with tissue specific defects in insulin sensitivity similar to human patients with type II diabetes mellitus. This animal model at six weeks of age is normoglycemic, but glucose intolerant (Clark JB, Palmer CJ, and Shaw WN: The diabetic Zucker fatty rat, Proceeding of the Society for Experimental Bioloqy and Medicine 173, 68-75, 1983; and Peterson RG, Shaw WN, Neel M, Little LA, Eichburg J: Zucker diabetic fatty rat as a model for non-insulin dependent diabetes mellitus. ILAR News 32:16-19, 1990.) Between seven and eight weeks of age, plasma glucose levels rise as the pancreatic beta cells lose their ability to secrete sufficient insulin to compensate for insulin resistance. In an attempt to maintain plasma glucose in normal range, the pancreas is forced to secrete increasing amount of insulin, resulting in hyperinsulinemia (Bue JM and Shaw WN: ZDF-Drt-fa rat: A new model with marked susceptibility to dietary manipulation. Diabetes 40:865A, 1991). The ZDF rat is usually studied between eight and nine weeks of age when it is hyperglycemic, hyperinsulinemic, and hyperlipemic, in an attempt to halt further decline of pancreatic function. The present study was carried out in six weeks old animals (prediabetic). The purpose of this study was to determine whether moxonidine treatment will prevent or delay onset of diabetes in the six weeks old prediabetic ZDF rat.

A number of interventions are capable of impacting the disease process in the ZDF rat, including agents that enhance peripheral insulin sensitivity (thiazolidinediones, TZDs), agents that act at the level of the pancreas glucagon like insulinotropic peptide (GLP-1), agents that affect intestinal absorption of glucose (acarbose), and agents with poorly defined mechanisms (metformin). These interventions also are effective in human NIDDM patients. As in humans, weight loss also improves insulin sensitivity.

To determine the impact of treatment with the substituted aminopyrimidines of Formula I on the progression of disease 6 week-old ZDF animals were grouped into the following categories, with 5 animals assigned to each group.
- Group 1: Control (5008 chow ad libitum)
- Group 2: Moxonidine (10 mg/kg/day)
- Group 3: Pair fed to correct for decrease in food consumption in 10 mg/kg/day group

Rats were housed one per plastic cage with bedding and had free access to water and Purina Formulalab 5008 Chow (except pair fed group). Because previous studies had indicated a reduction in food consumption with moxonidine treatment, body weight and food consumption were monitored daily, and a group of control animals was pair fed to moxonidine treated group. Blood samples were collected from the tail vein. Plasma glucose and insulin levels are monitored weekly.

Plasma glucose was measured by coupled hexokinase method on the IL Monarch System (Instrumentation Laboratory, Lexington, MA). Plasma insulin was determined with radioimmunoassay kit from Diagnostics Products Corporation (San Diego, CA) using rat insulin as a standard.

Plasma glucose and plasma insulin were monitored weekly. The results of these in vivo experiments are set forth below in Tables 1 and 2. The value in parenthesis following the plasma glucose value and plasma insulin value is the standard error of the mean.

| Effect of Moxonidine on Plasma Glucose (mg/dl) | | | |
|---|---|---|---|
| Moxonidine | | | |
| Day | Control | 10 mg/kg | Pair fed |
| 0 | 141.3 (3.9) | 145.6 (2.4) | 148.3 (2.4) |
| 7 | 141.1 (10.8) | 148.1 (13.8) | 129.4 (5.0) |
| 14 | 320.2 (38.8) | 166.1 (11.2) | 132.3 (11.5) |
| 21 | 436.8 (40.9) | 164.2 (8.0) | 245.1 (35.9) |
| 28 | 555.6 (19.8) | 168.0 (12.6) | 473.1 (16.7) |
| 35 | 574.5 (18.8) | 191.6 (8.2) | 489.6 (9.1) |
| 42 | 565.0 (18.3) | 179.8 (6.1) | 481.4 (11.9) |
| 49 | 536.6 (12.4) | 152.1 (8.7) | 482.0 (9.2) |
| 63 | 541.3 (24.7) | 168.4 (12.6) | 531.0 (24.9) |

| Effects of Moxonidine on Plasma Insulin (ng/ml) | | | |
|---|---|---|---|
| Moxonidine | | | |
| Day | Control | 10 mg/kg | Pair fed |
| 0 | 8.4 (1.1) | 10.9 (0.9) | 9.9 (1.3) |
| 7 | 19.0 (0.3) | 9.3 (1.0) | 14.1 (2.5) |
| 14 | 18.1 (1.5) | 12.7 (1.4) | 14.8 (2.5) |
| 21 | 13.8 (2.2) | 17.8 (1.2) | 20.5 (2.2) |
| 28 | 8.5 (1.8) | 23.4 (1.8) | 15.3 (3.3) |
| 35 | 6.3 (0.8) | 24.8 (1.4) | 12.2 (2.0) |
| 42 | 4.8 (0.5) | 20.6 (0.7) | 6.8 (0.9) |

The above data clearly demonstrates moxonidine's impact in preventing deterioration of glucose metabolism in a mammal having a genetically predisposed glucose metabolism abnormality in the absence of hyperglycemia.

A further study was carried out as described above, except that moxonidine treatment was reduced to 6.4 mg/kg/day for two days, followed by resumption of moxonidine treatment at 10 mg/kg/day for 13 days, and then stopping moxonidine treatment. Data on plasma glucose in mg/dl over the treatment period in days is reported below in Table 3.

| Moxonidine Effect on Plasma Glucose (mg/dl) | | | | | | | |
|---|---|---|---|---|---|---|---|
| Treatment period (days) | Control | Control sem | Mox. | Mox sem | pr fed | pr fed sem | Comment |
| 0 | 140.4 | 3.817853 | 141.5333 | 4.100705 | 141.1 | 3.993244 | mox @ 10 m/k/d |
| 7 | 187.4889 | 9.105252 | 152.8917 | 10.69197 | 101.8222 | 1.67174 | |
| 14 | 299.3694 | 46.70882 | 166.2528 | 5.754693 | 255.9278 | 34.40768 | |
| 21 | 405 | 58.78127 | 172.1556 | 17.00969 | 204.9278 | 18.56692 | Reduced mox dose to 6.4 m/k/d |
| 23 | 443.4333 | 27.22072 | 254.0889 | 38.84799 | 317.8556 | 48.35806 | mox @ 10 m/k/d |
| 28 | 459.6389 | 29.02029 | 251.4833 | 20.28488 | 422.4944 | 51.87904 | |
| 36 | 501.4239 | 20.33956 | 242.5944 | 42.08123 | 442.55 | 26.80212 | stopped mox treatment |
| 42 | 556.2778 | 21.15415 | 265.9556 | 42.19204 | 549.1667 | 25.56191 | 1 wk after mox is removed |
| 49 | 595.8 | 13.16921 | 410.425 | 46.99505 | 601.35 | 20.43749 | 2 wks after mox is removed |

The above data, Tables 1, 2 and 3, clearly demonstrates moxonidine's impact on preventing deterioration of glucose metabolism by intervening at one or more of the etiological cause(s) of glucose metabolism abnormalities.

## Claims

1. The use of a compound having the Formula 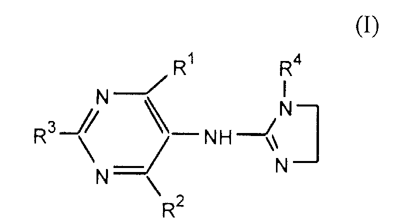 wherein R¹, R², and R³ are, independently, hydrogen, halogen, C₁-C₄ alkoxy, C₁-C₄ alkylthio, C₁-C₄ alkyl, or C₃-C₅-cycloalky;
R⁴ is hydrogen, formyl, acetyl, propionyl or butyryl;
provided that R¹, R² and R³ are not all hydrogen at the same time;
or a pharmaceutically acceptable salt or solvate thereof, in the preparation of a medicament useful for inhibiting glucose metabolism deterioration, in the presence of a glucose metabolism abnormality and in the absence of hyperglycemia.

2. The use of Claim 1 wherein
R¹ is chloro,
R² is methoxy,
R³ is methyl, and
R⁴ is hydrogen
or a pharmaceutically acceptable salt of solvate thereof.

3. The use of Claim 2 wherein said medicament is useful for treating a glucose metabolism abnormality selected from hyperinsulinemia, hypoinsulinemia in the presence of functioning beta cells, insulin resistance, impaired glucose tolerance and increased hepetic glucose output.

4. The use of Claim 2 wherein said medicament is useful for treating a previous glucose metabolism abnormality, or a potential glucose metabolism abnormality.
